# EUROPEAN PATENT APPLICATION

(11) **EP 2 520 578 A1**
(43) Date of publication of application: **07.11.2012**
(21) Application number: 12003426.9
(22) Date of filing: 04.05.2012
(51) Int. Cl.: C07D 501/12

(54) **Process for purification of cephalosporins**

(30) Priority: 06.05.2011 IN KO06302011
(71) Applicant: Lupin Limited, Mumbai - 400 098, Maharashtra (IN)
(72) Inventor: Dantu, Murali, Krishna, Mandideep-462046 District Raisen (M.P) (IN); Wankhade, Raju, Mandideep-462046 District Raisen (M.P) (IN); Pant, Brijesh, Mandideep-462046 District Raisen (M.P) (IN); Vejendla, Venkateswara, Rao, Mandideep-462046 District Raisen (M.P) (IN); Badoniya, Sourabh, Mandideep-462046 District Raisen (M.P) (IN)
(74) Representative: Ricker, Mathias

(57) **Abstract**

The present invention relates to process for purification of cephalosporins which comprises of i) conversion of cephalosporin to oxalate salt and ii) reacting the oxalate salt of cephalosporin with a base to obtain pure cephalosporin.

## Description

### Technical field of the Invention

The present invention relates to process of purification for cephalosporins which provides a simple and cost effective method for obtaining cephalosporins in high purity, which conforms to the pharmacopeial specifications.

### Back ground of the invention

Cephalosporin drugs such as cefpodoxime proxetil and cefditoren pivoxil are widely used for the treatment and prevention of various infectious diseases caused by pathogenic bacteria. Cefpodoxime proxetil is a third generation cephalosporin antibiotic for oral administration and has a broader antibacterial spectrum over the general gram positive and gram negative bacteria, especially against Streptococci, than other antibiotics for oral administration. The activity is primarily due to the cefpodoxime acid which is generated easily *in-vivo* by the hydrolysis of its proxetil ester. Cefpodoxime proxetil is specifically disclosed in US 4,486,425 and is represented by following structural formula:

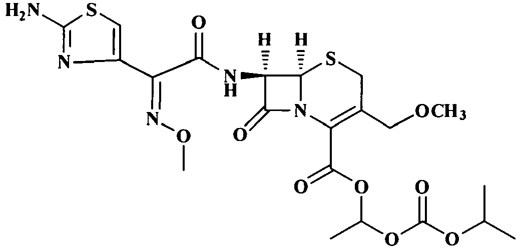

Cephalosporin antibiotics belonging to the class of 3-(2-substituted vinyl) cephalosporins have a very broad spectrum of antimicrobial activity. Cefditoren pivoxil, which belongs to this class, is highly active not only against a variety of gram-positive and gram-negative bacteria, but also against some resistant strains of bacteria. Due to the presence of vinyl group on the 3-position of the cephalosporin ring, cefditoren pivoxil is present in two isomeric forms, viz., the E- or Z-isomeric forms. The antimicrobial activity, however, resides primarily in Z-isomer while the E-isomer exhibits no significant antimicrobial activity.Cefditoren pivoxil is specifically disclosed in US 4,839,350 and is represented by following structural formula:

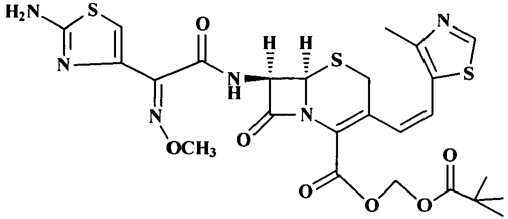

A number of methods have been outlined in U.S 4,486,425, US 4,482,710 and US 5,461,043 for the synthesis of the cefpodoxime esters. However, in each of these methods, esterification of the carboxylic acids of the cephem ring results in impurities which have to be removed using silica gel column chromatography.

PCT application WO 99/35149 describes the preparation of cefpodoxime proxetil with a focus on the adjustment of diastereoisomeric ratio of the two diastereoisomers of cefpodoxime proxetil in the product mixture. Although, the process does involve chromatographic techniques for isolation of products, but involves additional steps of protection and deprotection at the amino position of the thiazolyl moiety.

US 6,639,068 B1, relates to a method of preparing cefpodoxime proxetil having purity up to 99% using crown ethers. US 5,498,787 and KR 99/54751 use expensive quaternary ammonium salt phase transfer catalyst for preparation of pure cefpodoxime proxetil. WO 2004/060896 A1, teaches a process for the preparation of cefpodoxime proxetil free of impurities having a isomeric ratio ranging between 0.5 - 0.6 using methane sulphonic acid. US 7,045,618 B2 teaches an improved and cost effective process for the preparation of cefpodoxime proxetil having an assay up to 98% by solvent purification.

Impurities which are commonly present in cefpodoxime proxetil are as follows:

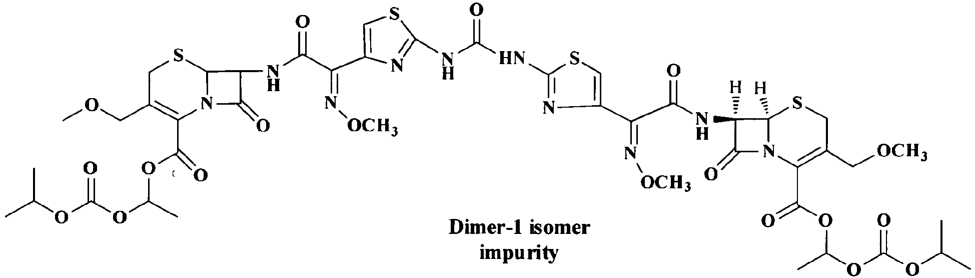

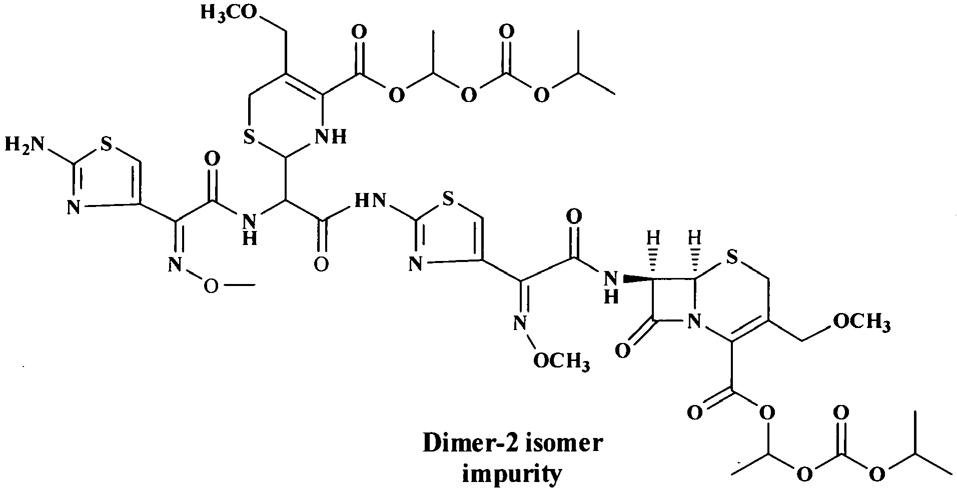

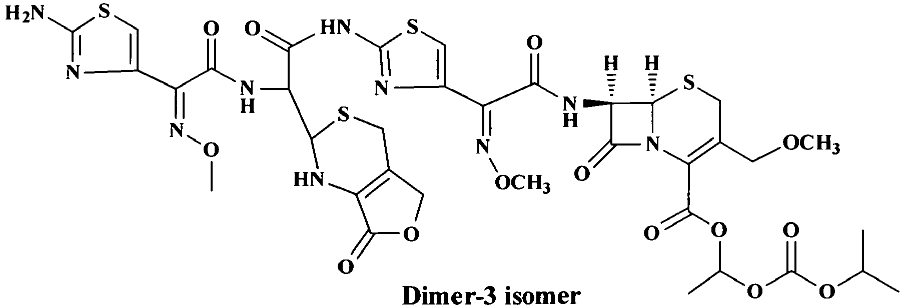

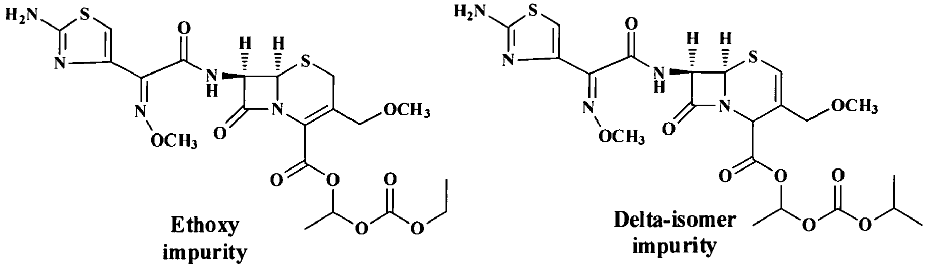

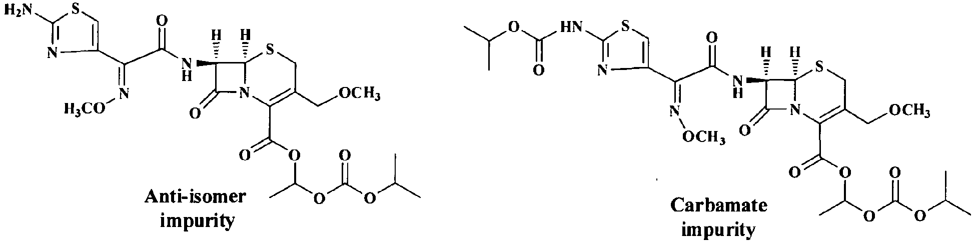

US 4,839,350 describes a process for preparing amorphous form of cefditoren pivoxil. This process is non-selective and giving more than 20% of unwanted E-isomer, which is then separated by means of column chromatography.

US 6,294,669 describes a crystalline substance of cefditoren pivoxil and process for preparing the same. The crystalline substance is described as having a purity of about 97 to 98%, typically 97.7%, which is believed to be not sufficiently pure for incorporation into a pharmaceutical composition due to the high amount of impurities present.

European Patent No. 175610 discloses a process for preparing cefditoren and its pharmaceutically acceptable salts and esters. The process is non-selective and yields more than 20 % of the undesirable E-isomer, which is then tediously separated by column chromatography.

U.S. Patent No. 6,288,223 discloses a process for the selective preparation of Z- isomer of 3-2-(substituted vinyl) cephalosporins. In this process, the reaction condition, as well as solvent system is selected so that the Z-isomer is selectively obtained without the formation of the E-isomer during the formation of the vinyl group. The process, however, generates about 4 to 5 % of the unwanted E-isomer while requiring separation to obtain the desired purity of the finished product. Also, it gives cefditoren pivoxil in low yield.

Impurities which are commonly present in cefditoren pivoxil are listed below:

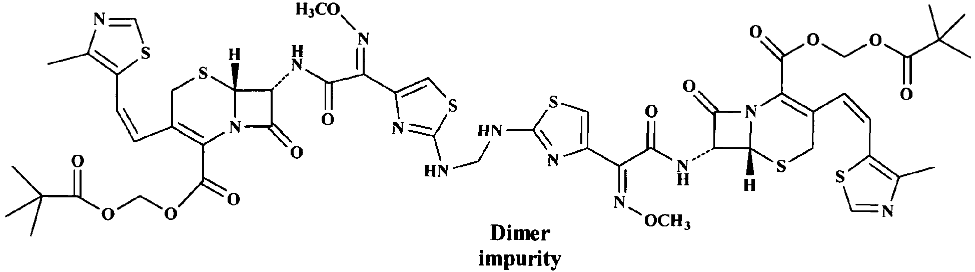

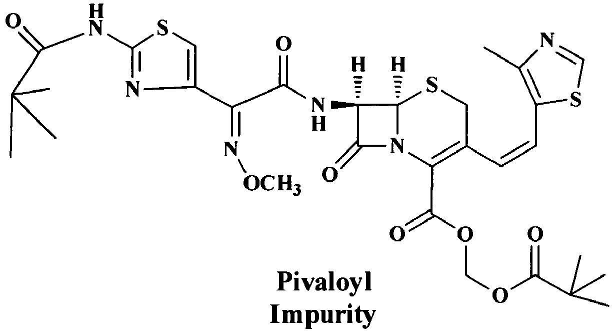

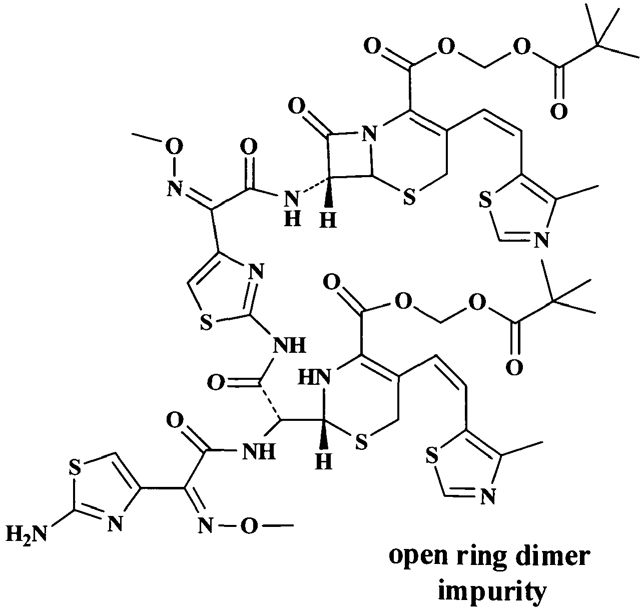

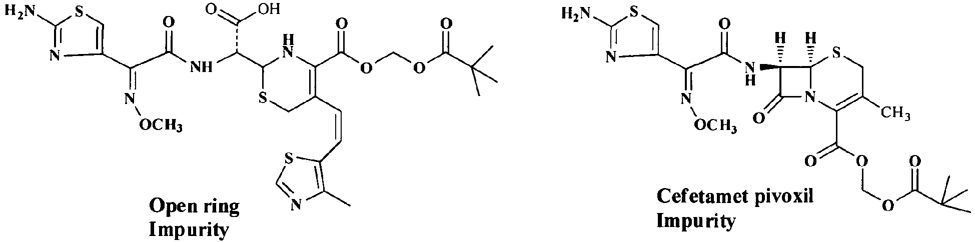

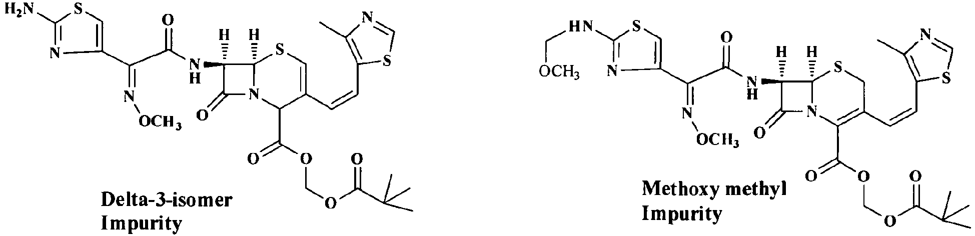

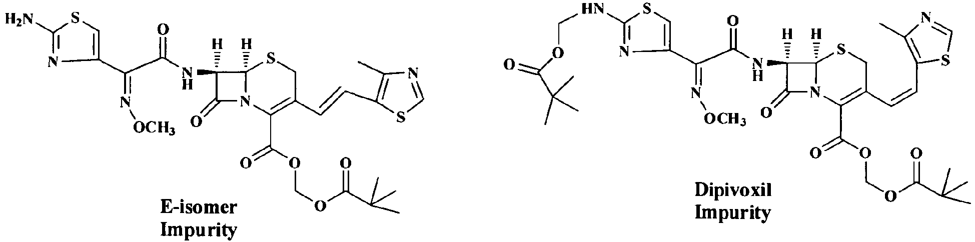

The methods employed in prior art for preparation and purification of cephalosporins suffer from following drawbacks:
1) Use of protection and deprotection of reactive functional group increases the number of steps and consequently the cost of the process.
2) Separation techniques like chromatography cannot be applied for large scale production.
3) Tedious multiple crystallization steps are required for obtaining a pure compound.
4) Difficult in maintaining the ratio of diastereomers R and S to conform to the pharmacopeial standards.
5) Difficult in maintaining the E/Z isomer ratio is difficult on industrial scale for obtaining the desired isomer.

Cephalosporin drugs are required in highly pure form because of the fear of unknown and potentially harmful effects of impurities. For purposes of patient safety, it is highly desirable to limit the amount of impurities present in any medicament administered to a patient. Considering the foregoing limitations, we undertook an investigation in our lab to identify a process, which yields a product with high purity and with less number of impurities. This would permit commercializing the production of highly pure cephalosporin drugs such as cefpodoxime proxetil and cefditoren pivoxil without any further purification, which in turn reduces the consumption of the solvents for purification, which directly has effect on the total cost of the process and is also eco-friendly.

### Object of the invention

The objective of the present invention is:
1) To provide an improved commercially viable process for the preparation of cephalosporins, this would be easy and cost effective to implement on industrial scale.
2) To obtain cephalosporin with high purity and better yield.
3) To provide an improved process for purification of cephalosporins, which avoids use of column chromatography for purification.

### Summary of the invention

The present invention provides an improved process for the preparation of pure cephalosporins of formula (I), 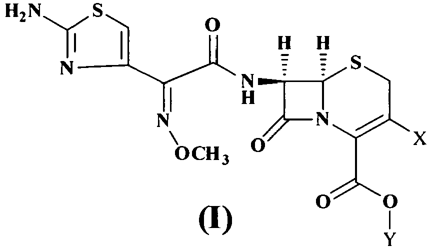 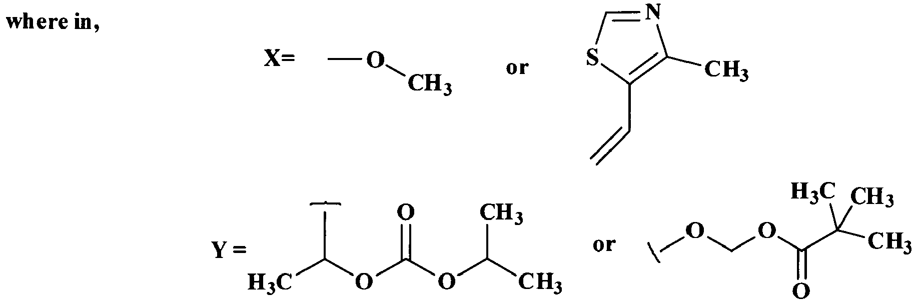 which comprises of :
i) conversion of cephalosporin to oxalate salt; and
ii) reacting the oxalate salt of cephalosporin with a base to obtain pure cephalosporin.

### Detail description of the invention

The present invention provides an improved process for preparation of pure cephalosporins of formula (I) 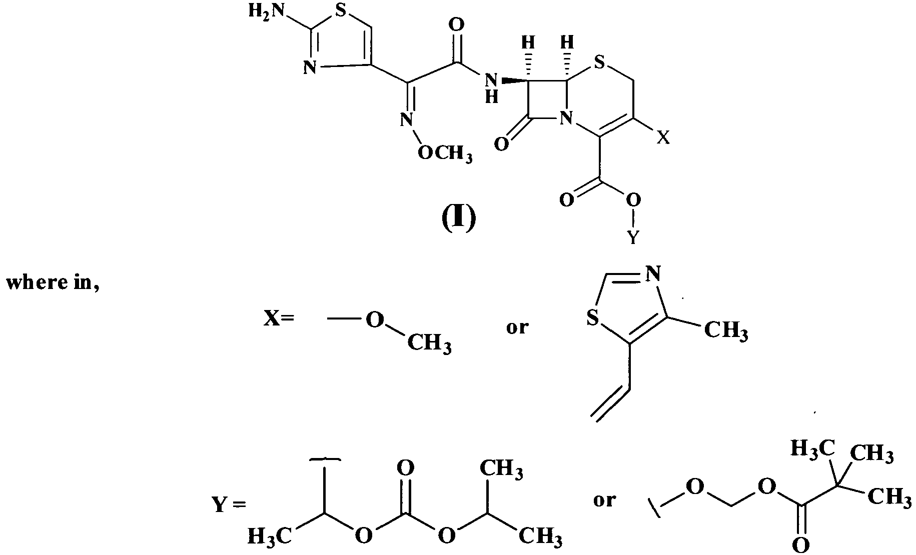 which comprises of:
(i) conversion of cephalosporin to oxalate salt; and
(ii) reacting the oxalate salt of cephalosporin with a base to obtain pure cephalosporin. The process of present invention is depicted in the following scheme:

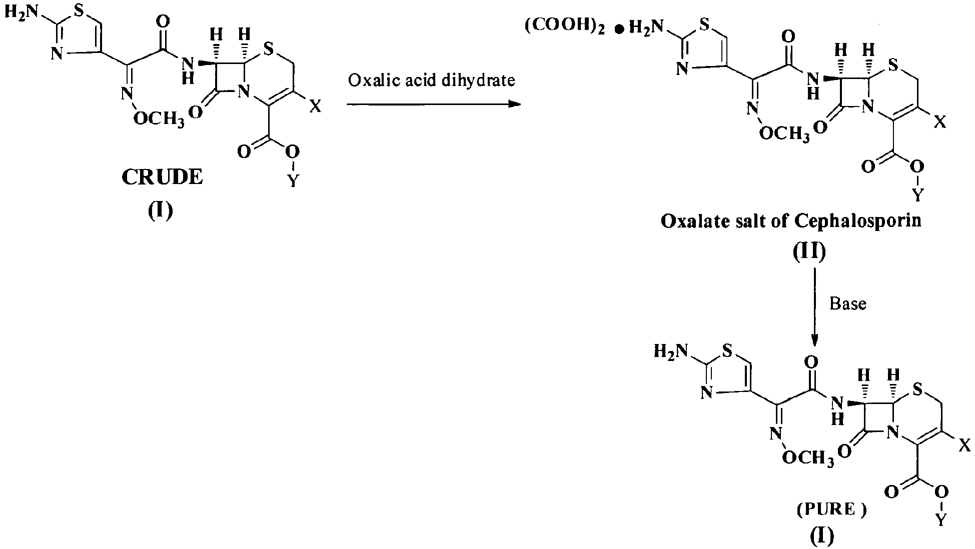

In one aspect of the present invention conversion of cephalosporin to oxalate salt is carried out by salt formation reaction with oxalic acid dihydrate.

The organic solvent employed for conversion of cephalosporin to its oxalate salt is selected from hydrocarbon solvents such as pentane, cyclopentane, hexane, cyclohexane, benzene, toluene; ethers such as dimethylether, diethyl ether, THF and dioxane. Preferably diethyl ether or cyclohexane is employed.

The ratio of solvent employed for the reaction with respect to cephalosporin is 1 to 50 volumes, more preferably 5 to 15 volumes, most preferably 8-10 volumes.

The molar ratio of oxalic acid dihydrate employed for the reaction with respect to cephalosporin is 0.1 to 10 molar equivalents, more preferably 1 to 3 molar equivalents, most preferably 1.5 molar equivalents.

The reaction temperature is in the range of 10-100°C, more preferably 15-40 °C, and most preferably 20-30 °C. The reaction mixture is stirred for 0.5 to 1.5 hours, preferably 1.0 hour. In another embodiment of the present invention, the oxalate salt of cephalosporin was reacted with suitable base to obtain pure cephalosporin.

The base employed for the reaction is an organic or inorganic base. The organic base is selected from triethylamine, diethyl amine. The inorganic base is selected from alkali metal carbonates such as sodium bicarbonate, sodium carbonate, potassium carbonate, potassium bicarbonate and ammonia. Preferably ammonia or sodium bicarbonate is used.

If the inorganic base such as sodium bicarbonate is employed then the pH of the above mixture is adjusted to 5.0 to 5.8 with sodium bicarbonate solution. The product obtained is filtered and dried under vacuum.

If the base such as ammonia is employed the solution of oxalate salt of cephalosporin is cooled, and organic solvent is added to the solution. The pH of the solution is adjusted to 5.0 to 5.8 with ammonical solution. The layers are separated and the aqueous layer is extracted with organic solvent. The organic layers are combined and subjected to charcolisation. The resultant solution is added to cyclohexane to complete the crystallization. The obtained product is filtered and washed with water and dried under vacuum.

The organic solvent employed in step (ii) is selected from methanol, ethanol, acetone, ethyl acetate butyl acetate, methyl ethyl ketone, dichloromethane. Preferably ethyl acetate is employed.

The product is isolated by conventional techniques known in prior art such as centrifugation, filtration etc.

The invention is further illustrated by way of following examples which should not be construed as limiting to the scope of the invention.

### Example-1

### Preparation of oxalate salt of cefpodoxime proxetil:

100 gm (0.2341 mole) of cefpodoxime acid was dissolved in 1400ml of dimethyl acetamide. The solution was cooled to -10°C. 34.94gm (mole 0.2295) of DBU was added to the solution followed by the addition of 63.44gm (mole 0.2459) of 1-iodoethyl isopropyl carbonate at -8° to -10°C. The reaction mass was stirred for 30-90 minutes and was warmed to 0-5°C. A solution of 6.5 gm of sodium hydrosulfite in 50ml water was added to the reaction mass, followed by addition of 1400ml ethyl acetate and 800ml DM water. The resulting mixture was stirred and 3.5% hydrochloric acid (200ml) was added and stirred. The organic layer was separated and pH of the aqueous layer was adjusted to 5.0 with 6% sodium bicarbonate solution at 0-5°C and extracted twice with 700ml ethyl acetate. The layers were separated. Organic layer was combined with 5% aqueous sodium thiosulphate (500 ml) followed by 2.4% aqueous sodium bicarbonate solution (500 ml) and finally with water (500 ml).

The resulting ethyl acetate layer was subjected to charcolisation, filtered and the carbon bed was washed with 200 ml of ethyl acetate. The combined organic layer was concentrated under reduced pressure. 100ml of cyclohexane was added under stirring followed by 44.26gm (moles 0.3512) of oxalic acid dihydrate, and further stirred at 20-25°C. 1200ml of cyclohexane was further added and stirred for 60 minutes. The product was filtered at 5-10°C and dried under vacuum at 35°C. The % yield of the product is listed in the following table.

| Proxetil content in solution after reaction and work up | | | Oxalate salt (Isolated) | | | Yield of Proxetil (%) |
|---|---|---|---|---|---|---|
| Solution Weight (gm) | Proxetil content (%) | Net proxetil (gm) | Wt of Oxalate Salt (gm) | Proxetil Content | Net proxetil (gm) | |
| 224 | 36.62 | 82.02 | 119.50 | 68.58 | 81.95 | 99.91 |
| 300 | 26.61 | 79.83 | 118.20 | 67.15 | 79.37 | 99.42 |

Yield and purity of cefpodoxime proxetil is calculated based on content of cefpodoxime proxetil in oxalate salt.

The above experiment can also be repeated with the same experimental conditions but by using n-hexane and ether as solvents for preparation of oxalate salt of cefpodoxime proxetil.The % yield of the product is listed in the following table.

| Cefpodoxime Acid (gm) | Oxalic acid dihdrate (gm) | Solvent | Oxalate Salt Obtained | | | | | Yield of Proxetil (%) |
|---|---|---|---|---|---|---|---|---|
| | | | Oxalate salt (gm) | Proxetil content (%) | Oxalic acid (%) | Water content (%) | Net Proxetil (gm) | |
| 60 | 26.57 | Cyclohexane | 90.6 | 67.63 | 22.9 | 6.49 | 61.02 | 77.93 |
| 20 | 8.85 | n-Hexane | 29 | 67.64 | 25.6 | 4.93 | 19.61 | 75.10 |
| 20 | 8.85 | Diisopropyl ether | 29.8 | 55.37 | 31.2 | 8.38 | 16.50 | 63.21 |

Yield and purity of cefpodoxime proxetil is calculated based on content of cefpodoxime proxetil in oxalate salt.

### Example-2

### Preparation of pure cefpodoxime proxetil:

50 gm (mole 0.0767) of oxalate salt obtained from the above example was dissolved in water (500 ml) at 18-20°C. The pH of mixture was adjusted to 5.0 to 5.8 with 5% sodium bicarbonate solution. Then the mixture was stirred for 60 minutes and the product obtained was filtered. The product was washed with water and dried under vacuum at 40°C to obtain pure cefpodoxime proxetil having an isomer ratio of (R/R+S) = 0.52. The % yield of the product and the impurity data are listed in the following table.

| Yield | | Impurities (%) | | | | | | | | Assay (%) | R/R+S |
|---|---|---|---|---|---|---|---|---|---|---|---|
| gm | (%) | Delta isomer | Anti Isomers | | Dimer-1 Isomers | | Dimer-2 Isomers | | Total Impurity | | |
| | | | A | B | I | II | I | II | | | |
| 39 | 95.47 | 1.0 | 0.15 | 0.14 | 0.03 | 0.01 | BDL | 0.03 | 3.52 | 96.1 | 0.53 |
| 39.5 | 97.17 | 1.21 | 0.08 | 0.08 | 0.03 | 0.02 | 0.03 | 0.04 | 3.15 | 97.1 | 0.54 |

Yield and purity of cefpodoxime proxetil is calculated based on content of cefpodoxime proxetil in oxalate salt. In the impurity data given above, I and II represent conformational analogues of the respective impurities.

### Example-3

### Preparation of pure cefpodoxime proxetil:

45 gm of oxalate salt of cefpodoxime proxetil was added to water (450 ml) and the solution was cooled to 15-18°C. Ethyl acetate (157.5 ml) was added to the solution and pH of solution was adjusted to 5.0 to 5.8 with 12.5% aqueous ammonical solution at 18-20°C. The layers were separated and the aqueous layer was extracted with ethyl acetate (45 ml). The two organic layers were combined and subjected to charcolisation. The resultant solution was added to cyclohexane (675 ml) to crystallize the product. The product was filtered, washed with water and dried under vacuum to afford pure cefpodoxime proxetil having an isomer ratio of (R/R+S) = 0.52. The % yield of the product and the impurity data are listed in the following table.

| Yield | | Impurities (%) | | | | | | | | Assay (%) | R/R+S |
|---|---|---|---|---|---|---|---|---|---|---|---|
| gm | (%) | Delta isomer | Anti Isomers | | Dimer-1 Isomers | | Dimer-2 Isomers | | Total Impurity | | |
| | | | A | B | I | II | I | II | | | |
| 28.26 | 89.28 | 0.73 | 0.07 | 0.03 | 0.03 | 0.01 | 0.04 | 0.03 | 1.81 | 95.9 | 0.52 |
| 24.35 | 86.30 | 0.71 | 0.07 | 0.03 | 0.02 | 0.02 | 0.05 | 0.04 | 2.02 | 96.8 | 0.52 |

Yield and purity of cefpodoxime proxetil is calculated based on content of cefpodoxime proxetil in oxalate salt. In the impurity data given above, I and II represent conformational analogues of the respective impurities.

### Example-4

### Preparation of pure cefpodoxime proxetil:

100 gm (moles 0.1534) of oxalate salt of cefpodoxime proxetil was added to water (1000 ml) and cooled to 15-18°C. Ethyl acetate (400 ml) was added to the solution and the pH of the solution was adjusted to 4.3 to 5.0 with aqueous ammonical solution (12.5%). The layers were separated and the aqueous layer was extracted with ethyl acetate. The organic layers were combined and subjected to charcolisation. The combined organic layer was concentrated under reduced pressure till thick mass was obtained. Methanol (140 ml) was added and the obtained solution was added to water (1750 ml) at 5-10°C to complete the crystallization. The product obtained was filtered, washed with water and dried under vacuum at 35-40°C to give pure cefpodoxime proxetil having an isomer ratio of (R/R+S) = 0.52.

For further purification 65gm (0.117 moles) of the product was dissolved in 162.5 ml of methanol at 5 to 10°C and was subjected to charcolisation. The resulting mixture was stirred, filtered and the carbon bed was washed with 97.5 ml of methanol. The filtrate was added slowly to 1040 ml of water and stirred. The product was filtered, washed with 910 ml of water and dried under reduced pressure at 40°C. The yield of the product and the impurity data are listed in the following table.

| Yield | | Impurity (%) | | | | | | | | Assay (%) | R/R+S |
|---|---|---|---|---|---|---|---|---|---|---|---|
| gm | (%) | Delta isomer | Anti Isomers | | Dimer-1 Isomers | | Dimer-2 Isomers | | Total Impurity | | |
| | | | A | B | I | II | I | II | | | |
| 46.46 | 86.2 | 0.75 | 0.04 | 0.03 | 0.02 | 0.01 | 0.04 | 0.03 | 1.67 | 100.3 | 0.53 |
| 46.49 | 84.6 | 0.73 | 0.07 | 0.04 | 0.01 | 0.01 | 0.03 | 0.02 | 1.68 | 99.9 | 0.53 |
| 46.09 | 82.1 | 0.61 | 0.03 | 0.02 | 0.01 | 0.01 | 0.03 | 0.02 | 1.38 | 100.0 | 0.53 |

Yield and purity of cefpodoxime proxetil is calculated based on content of cefpodoxime proxetil in oxalate salt. In the impurity data given above, I and II represent conformational analogues of the respective impurities.

### Example-5

### Preparation of pure cefpodoxime proxetil:

30 grams of oxalate salt of cefpodoxime proxetil was added to acetone (75 ml) and the solution was cooled to 5-10°C. The pH of the mixture was adjusted to 5 to 5.8 with 25 % aqueous ammonia solution. The resulting mixture was stirred at 5 to 10°C and the solid obtained was filtered and washed with acetone. The filtrate was subjected to charcolisation and the carbon bed was washed with 45 ml of acetone. The filtrate was added to 840 ml of water at 5-10°C to obtain the product. The slurry was filtered, washed with 420 ml of water and dried under vacuum.

The other water miscible solvents such as ethanol or methanol may also be used. In place of ammonia solution, other base like triethylamine, diethyl amine or methanolic ammonia solution may also be used. The yield of the product and the impurity data are listed in the following table:

| Solvent Used | Base used | Yield | | Impurity (%) | | | | | | | | Assay (%) | R/R+S |
|---|---|---|---|---|---|---|---|---|---|---|---|---|---|
| | | gm | (%) | Delta isomer | Anti Isomers | | Dimer-1 Isomers | | Dimer-2 Isomers | | Total Impurity | | |
| | | | | | A | B | I | II | I | II | | | |
| MeO H | TEA | 17.4 | 84.4 | 1.30 | 0.02 | 0.02 | 0.02 | 0.01 | 0.01 | BDL | 2.63 | 98.9 | 0.52 |
| MeO H | MeO H+ NH₃ | 12.0 | 61.41 | 0.68 | 0.03 | 0.02 | 0.25 | BDL | BDL | 0.01 | 3.24 | 98.9 | 0.52 |
| ME K | Aq.N H₃ | 18.9 | 97.2 | 1.16 | 0.02 | 0.01 | 0.01 | 0.01 | 0.03 | 0.03 | 2.30 | 98.1 | 0.52 |

Yield and purity of cefpodoxime proxetil is calculated based on content of cefpodoxime proxetil in oxalate salt. In the impurity data given above, I and II represent conformational analogues of the respective impurities.

### Example-6

### Preparation of oxalate salt of cefditoren pivoxil:

16.5gm (0.03 mole) of sodium salt of cefditoren was dissolved in 99ml of DMF and the mixture was cooled to -25 to -30°C. 0.79 gm (0.009 mole) of sodium bicarbonate was added to the reaction mixture followed by 9.05 gm (0.037 mole) of 1-iodomethyl pivolate at -25 to - 30°C. The reaction mass was stirred. After the completion of reaction, aqueous sodium hydrosulfite ( 1.07gm dissolved in 8.25 ml water), 231 ml of ethyl acetate and 132 ml of water was added at 0-5°C. The resulting mixture was stirred and 33 ml (3.5 % w/v) of dilute hydrochloric acid was added. The organic layer was separated. The pH of the aqueous layer was adjusted to 5.0 by 6% sodium bicarbonate solution and extracted with 115 ml of ethyl acetate. The organic layers were combined, washed with 82.5 ml of 5 % aqueous sodium thiosulphate followed by 82.5 ml of 2.4 % aqueous bicarbonate solution and finally by 82.5 ml of water. The resulting ethyl acetate layer was subjected to charcolisation and filtered. The carbon bed was washed with 33 ml of ethyl acetate. 5.61 gm (moles 0.0445) of oxalic acid dihydrate was and 198 ml of cyclohexane was added, stirred and the product obtained was filtered and dried under reduced pressure.
Yield = 20.45 gm
% yield = 77.1%

### Example-7

### Preparation of pure cefditoren pivoxil:

10 grams of oxalate salt of cefditoren pivoxil was added to 100 ml of water at 18-20°C and the pH of mixture was adjusted to 6.0 to 6.5 by 8% aqueous solution of sodium bicarbonate. The aqueous layer was extracted with dichloromethane (50ml), organic layer was separated and concentrated up to 1-2 volume under reduced pressure. 40ml of ethyl acetate was added to the residue with stirring at 5-10°C. The product obtained was filtered, washed with 10ml chilled ethyl acetate and dried under reduced pressure. The yield of the product and the impurity data are listed in the following table:

| yield | | Impurity (%) | | | | | | | | | | Assay (%) |
|---|---|---|---|---|---|---|---|---|---|---|---|---|
| gm | (%) | Openring | Cefta met pivoxil | Delta isomer | Met hoxymethyl | E-isomer | Dipivoxil | Pivaloyl | Dimer | Ring opening dimmer | Total impurity | |
| 6 | 86 | 0.07 | BDL | 0.36 | 0.05 | 0.5 | 0.16 | 0.05 | 0.72 | 0.16 | 2.4 | 98.7 |
| 5.8 | 85 | 0.07 | BDL | 0.36 | 0.05 | 0.61 | 0.16 | 0.06 | 0.74 | 0.16 | 2.5 | 99.1 |

| | | | | | | | | | | | | |
|---|---|---|---|---|---|---|---|---|---|---|---|---|
| BDL= below detection level; Yield and purity of cefditoren pivoxil is calculated based on content of cefditoren pivoxil in oxalate salt. | | | | | | | | | | | | |

## Claims

1. An improved process for the preparation of pure cephalosporins of formula (I), 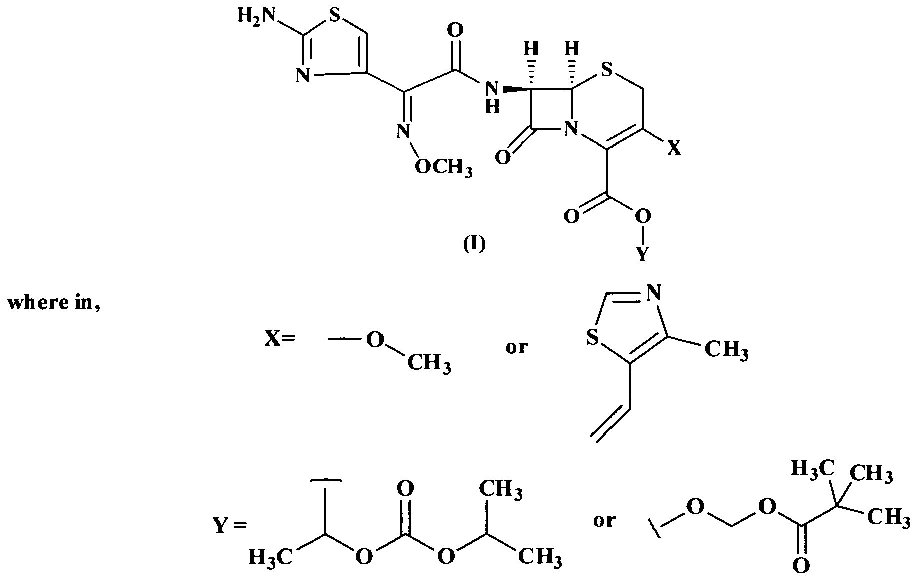 which comprises of :
(i) conversion of the said cephalosporin to the oxalate salt; and
(ii) reacting the oxalate salt of the said cephalosporin with a suitable base to obtain pure cephalosporin.

2. The process according to claim 1, wherein the solvent used in step (i) is selected from pentane, hexane, benzene, cyclohexane, cyclopentane , toluene, dimethyl ether, diethylether, THF and dioxane.

3. The process according to claim 2, wherein the solvent is cyclohexane or diethylether.

4. The process according to claim 1, wherein the base employed in step (ii) is organic or inorganic base.

5. The process according to claim 4, wherein the organic base employed in step (ii) is selected from triethylamine or diethyl amine.

6. The process according to claim 4, wherein the inorganic base employed in step (ii) is selected from NH₃, sodium carbonate, potassium carbonate, sodium bicarbonate and potassium bi carbonate.

7. The process according to claim 4, wherein the base is NH₃ and sodium bicarbonate.

8. The process according to claim 1, wherein the molar ratio of oxalic acid dihydrate employed for the reaction with respect to cephalosporin is 0 to 5.

9. The process according to claim 1 wherein, the solvent employed in step (ii) is selected from methanol, ethanol, acetone, ethyl acetate, butyl acetate, methyl ethyl ketone, and dichloromethane.

10. The process according to claim 9, wherein the solvent is selected from ethyl acetate and dichloromethane.

11. The process according to claim 1, wherein the cephalosporins are selected from cefpodoxime proxetil and cefditoren pivoxil.

12. The process according to claim 1 wherein, the diastereomeric ratio of (R/R+S) of pure cefpodoxime proxetil is between 0.5 to 0.6.
